# EUROPÄISCHE PATENTSCHRIFT

(11) **EP 3 280 807 B1**
(45) Veröffentlichungstag und Bekanntmachung des Hinweises auf die Patenterteilung: **09.10.2019**
(21) Anmeldenummer: 16713366.9
(22) Anmeldetag: 24.03.2016
(51) Int. Cl.: C12P 5/02, B01D 53/85, B01D 53/62, B01D 53/84, E21B 41/00, F17C 1/00

(54) **VERFAHREN ZUR HYDROGENOTROPHEN METHANOGENESE VON H2 UND CO2 ZU CH4**
METHOD FOR HYDROGENOTROPHIC METHANOGENESIS OF H2 AND CO2 TO CH4
PROCÉDÉ DE MÉTHANOGENÈSE HYDROGÉNOTROPHES DE H2 ET CO2 EN CH4

(30) Priorität: 26.03.2015 EP 15161055
(43) Veröffentlichungstag der Anmeldung: 14.02.2018
(73) Patentinhaber: RAG Austria AG, 1015 Wien (AT)
(72) Erfinder: MITTEREGGER, Markus, 1015 Wien (AT); BAUER, Stephan, 1015 Wien (AT); LOIBNER, Andreas P., 3012 Wolfsgraben (AT); SCHRITTER, Johanna, 3072 Neulengbach (AT); GUBIK, Alexander, 1015 Wien (AT); BACKES, Diana, 4102 Binningen (CH); PICHLER, Markus, 1015 Wien (AT); KOMM, Robert, 1030 Wien (AT); BRANDSTÄTTER-SCHERR, Kerstin, 1150 Wien (AT)
(74) Vertreter: Sonn & Partner Patentanwälte
(86) Internationale Anmeldenummer: PCT/EP2016/056521
(87) Internationale Veröffentlichungsnummer: WO 2016/151078

(56) Entgegenhaltungen:
- EP-A1- 0 963 780
- WO-A1-2008/128331
- WO-A1-2012/110256

## Beschreibung

Die vorliegende Erfindung betrifft ein Verfahren zur hydrogenotrophen Methanogenese von H₂ und CO₂ zu CH₄ durch Einbringung eines Gasgemisches aus Wasserstoff, Kohlendioxid und mindestens 10 % Erdgas in eine Gaszone eines untertägigen Speichers und Lagerung in Gegenwart von methanogenen Mikroorganismen.

Um die tageszeit- bzw. wettersituationsabhängige Verfügbarkeit erneuerbarer Energiequellen, wie Wind und Sonne, auszugleichen und dem Strombedarf anpassen zu können, werden Speicher für die erneuerbaren Energien benötigt. In beispielsweise Starkwindzeiten kann so überschüssig produzierte Energie gespeichert und später, beispielsweise in Zeiten erhöhten Strombedarfs, genutzt werden oder anderen energetischen Gasanwendungen zugeführt werden.

Energiespeicher dienen der Speicherung von Energie zur späteren Nutzung. Ist die Speicherung einer Energieform wegen technischer Probleme, ungenügender Kapazität oder Stillstandsverlusten ungünstig, wird diese Energieform in eine andere, für die Speicherung geeignetere Energieform umgewandelt und dann gespeichert, wobei im Bedarfsfall die Energie dann wieder zurückgewandelt werden kann. Ein Beispiel ist die Wandlung chemischer Energie (Brennstoff) in thermische Energie (Wärme) oder die Wandlung von elektrischer Energie (Strom) in chemische Energie (Brennstoff) oder thermische Energie (Wärme). Sowohl bei der Speicherung als auch bei der Energieumwandlung treten immer Verluste auf.

Elektrische Energie kann man nur schwer direkt speichern, in der Regel ist es erforderlich, die Energie in eine andere Energieart umzuwandeln und bei Bedarf zurück zu wandeln.

Dabei ist in den letzten Jahren der Power to Gas Ansatz entwickelt und in ersten Demonstrationsanlagen gezeigt worden, bei dem elektrische Energie in Gase umgewandelt und somit leichter speicherbar gemacht wird. Beispielsweise ist in Falkenhagen (Deutschland) eine Anlage errichtet worden, in welcher mittels Windstrom Wasser elektrolytisch in Wasserstoff und Sauerstoff zerlegt und der Wasserstoff dann in das lokale Erdgasnetz einspeist wird. Eine weitere Anlage in Werlte (Deutschland) zerlegt ebenfalls mittels Strom Wasser in Wasserstoff und Sauerstoff, wobei dann in einem weiteren katalytischen Prozessschritt aus Wasserstoff und Kohlendioxid Methan entsteht, das wiederum in die Erdgasinfrastruktur eingespeist wird.

Wasserstoff bildet in einem Anteil von 4 bis 75 Vol.-% mit Luft ein entzündliches Gemisch, ein explosives Gemisch (Knallgas) bildet Wasserstoff erst bei einem Anteil ab 18 Vol.-%. Weil Wasserstoff das leichteste von allen Elementen ist, verflüchtigt es sich in offener Umgebung, bevor es ein explosives Gemisch bilden kann, oder es brennt in heißen Umgebungen bereits bei der Konzentration von 4 Vol.-% (wenn im Rahmen der vorliegenden Beschreibung bzw. der Patentansprüche im Zusammenhang mit Gasgemischen eine Prozentangabe erfolgt, so sind damit immer Volumenprozent gemeint, bei Feststoffen und Flüssigkeiten sind Masseprozent gemeint, sofern nicht anders angegeben).

Die wohl bekannteste und verbreitetste Speicherform von Wasserstoff ist die so genannte Druckgasspeicherung und basiert auf Druckbehältern, wie beispielsweise Gasflaschen. Die Speicherung erfolgt dabei in geeigneten Behältern unter Drücken von etwa 200 bis 800 bar, bekannt sind auch schon Druckbehälter mit einem Speicherdruck von bis zu 1200 bar. Durch die erforderliche Stabilität der Druckbehälter liegt die Speicherdichte bei etwa 1 kg Wasserstoff in 70-80 kg Behältermasse, was die Druckgasspeicherung für den Transport höchst unökonomisch macht. Weltweit wird daher nur rund 1 % des produzierten Wasserstoffs in dieser Form gespeichert und transportiert.

In der Erdgasindustrie ist die Speicherung von Erdgas in Untertagespeichern Stand der Technik. Hier wird zwischen natürlichen Speicherformationen, wie ausgeförderten Öl und Gaslagerstätten sowie Aquiferen, und künstlichen Hohlräumen, wie Salzkavernen und Bergbaustollen, unterschieden. Die natürlichen Speicherformationen werden auch als Porenspeicher bezeichnet, da hier das Gas direkt in den Gesteinsporen gelagert wird.

Einen Indikator für die Speicherung lagerstättenfremder Gase bietet die Stadtgasspeicherung. Beispielsweise betreiben die Stadtwerke Kiel seit 1971 eine 32.000 m³ Gaskaverne für die Speicherung von Stadtgas, welches einen bis zu 50%igen Wasserstoffanteil hat. Die Kaverne liegt in einer Tiefe von 1330 m und die Speicherung erfolgt bei einem Druck von 80-160 bar, wobei die Gasverluste nur bei etwa 1 bis 3 % des Speichervolumens pro Jahr liegen. Entgegen landläufiger Meinung ist Stadtgas allerdings nicht direkt mit der Wasserstoffspeicherung oder der Biogasgenerierung vergleichbar, da vor allem das im Stadtgas enthaltene Kohlemonoxid eine weitere hochreaktive Komponente ist, die weitere chemische Prozesse hervorruft. Es gibt allerdings auch Erfahrungswerte aus der industriellen Wasserstoffspeicherung in Teeside (Großbritannien) und Bakniev (Russland), die als Vergleich herangezogen werden können. Zu guter Letzt kann auch noch auf Erfahrung aus der "CO2 supported EOR" (Enhanced Oil Recovery) zurückgegriffen werden.

Dementsprechend kann beim Verhalten von Wasserstoff im Untergrund bzw. bei dessen Speicherung im geologischen Untergrund gemäß Stand der Technik also auf langjährige praktische Erfahrung zurückgegriffen werden

Die Beimischung von Wasserstoff zu Erdgas unterliegt der Erdgasnorm, welche zurzeit dezentral in verschiedenen Ländern verschiedene Konzentrationen an Wasserstoff zulässt. In Österreich definiert beispielsweise die ÖVGW-Richtlinie G31 einen Wasserstoffanteil von maximal 4 % in jedem Teil des Erdgasnetzes. Befindet sich in der Nähe des Einspeisepunktes eine Erdgas-Tankstelle, so darf der Wasserstoffanteil nur maximal 2 % betragen. In Deutschland sind laut Arbeitsblatt DVGW-G 260 "Gasbeschaffenheit" Wasserstoffeinspeisungen im einstelligen Prozentbereich zulässig, wobei das tatsächliche Limit immer in einer Einzelfallbetrachtung ermittelt werden muss.

CO₂ wirkt in der Atmosphäre als Treibhausgas und gilt als die Hauptursache der globalen Erwärmung. Die Abtrennung von CO₂ beispielsweise aus den Abgasen von Kraftwerken kann mit unterschiedlichen Verfahren erfolgen, z.B. nach der Verbrennung in einer CO₂-Wäsche des Abgases, Abtrennung von CO₂ nach Kohlevergasung oder Verbrennung in Sauerstoffatmosphäre. Zur Speicherung von beispielsweise durch Auswaschen oder Abtrennen gewonnenem CO₂ wird unter anderem die geologische Speicherung vorgeschlagen, beispielsweise in geologischen Formationen, wie ausgeförderte Erdöl- und Erdgaslagerstätten, sowie in tiefen salzwasserführenden Grundwasserleitern (Aquifere). Die meisten Forscher favorisieren dabei eine Lagerung des CO₂ in tiefen Sedimentschichten, deren Poren mit Salzwasser gefüllt sind. Ab ca. 800 m Tiefe treten Drücke auf, bei denen das eingebrachte CO₂ so verdichtet ist, dass es im überkritischen Zustand bleibt. Damit ein erneutes Zutagetreten des Kohlendioxids praktisch ausgeschlossen ist, müssen diese Schichten durch eine undurchlässige Deckschicht abgedeckt sein. Durch den dort herrschenden Druck besitzt das CO₂ eine etwa so große Dichte wie das Salzwasser, wodurch es dieses aus den Poren verdrängen kann und damit Platz für das verpresste CO₂ geschaffen wird. Das Salzwasser wird dabei in der Lagerstätte lateral zur Injektionsstelle verdrängt. Die laterale Ausdehnung der Druckanomalie kann vielfach größer sein, als die Verbreitung des CO₂ in einem Aquifer. Werden zur Verpressung von CO₂ und zur Verdrängung von Salzwasser Drücke verwandt, die deutlich über dem Formationsdruck und der Zugspannung des Gesteins liegen, so können auch induzierte Erdbeben auftreten, die im Einzelfall auch zu Erschütterungen führen können, die über der Fühlbarkeitsgrenze liegen. Die unterirdische Lagerung von CO₂ ist daher problembehaftet, darüber hinaus steht die Lagerung bei der Nutzung tiefer Aquifer im Wettbewerb mit anderen Nutzungen, beispielsweise der Nutzung dieser Aquifer zur nachhaltigen Stromerzeugung aus Geothermie. Auch ist die Speicherfähigkeit von Aquiferen begrenzt.

Die EP 0 963 780 A1 offenbart ein Verfahren, bei welchem Wasserstoff und CO₂ in Anwesenheit von methanogenen Bakterien (richtig Mikroorganismen) in einem Porenspeicher gelagert werden. Aus dem Dokument ist somit bekannt, CO₂ mit bekannten technischen Mitteln aus Verbrennungsabgasen abzutrennen und unterirdisch zu speichern. Zu dem CO₂ können auch H₂ in reiner Form oder z.B. in Form von Ammoniak, methanogene "Bakterien", Bakteriensubstrat, Katalysatoren und/oder Inhibitoren zugesetzt werden, damit während der unterirdischen Speicherung das CO₂ zu CH₄ reduziert wird. Dabei wird das mit bekannten Mitteln aus einem Abgas abgetrennte, gereinigte, verflüssigte und getrocknete CO₂ über eine Fernleitung pumpbar gemacht und in einen benachbarten Aquifer, künstlicher unterirdischer Hohlraum oder eine Erdgas-bzw. Erdöllagerstätte eingepresst, wobei in den ersten beiden Fällen eine Impfung der geologischen Struktur durch entsprechende Bakterienkulturen und dazugehöriges organisches Substrat vorausgeht, im dritten Fall sind die methanogenen Bakterien sowie organisches Substrat bereits natürlich vorhanden. Wenn dieser Lehre gefolgt wird, bildet sich in der Lagerstätte durch Lösen des eingepressten CO₂ in Wasser Kohlensäure, wodurch zumindest in der Umgebung der Einpresssonde der pH auf etwa 4 abfällt, was die methanogenen Mikroorganismen inaktiv werden lässt. Abgesehen davon kommt es durch den niedrigen pH auch zu einer irreparablen Schädigung der in der Lagerstätte befindlichen Karbonatgesteine. Die gebildete Kohlensäure löst das Karbonatgestein einfach auf, wodurch die Stabilität des Porenspeichers gefährdet werden könnte. Darüber hinaus wird keine Durchmischung von Erdgas, Wasserstoff und CO₂ vorgesehen, wodurch die methanogenen Mikroorganismen weiter gestresst werden.

Die WO 2008/128331 A1 offenbart ein Verfahren, bei welchem in einem unterirdischen Lager Wasserstoff und CO₂ mittels methanogener Mikroorganismen zu Methan umgesetzt werden. CO₂ und Wasserstoff werden dem Lager zugeführt.

WO 2012/110256 offenbart ein Verfahren zur hydrogenotrophen Methanogenese von Wasserstoff und Kohlendioxid zu Methan mit methanogenen Mikroorganismen. Das Lagern und Umsetzen in unterirdischen Speichern wird nicht genannt.

Mikrobiell wird Methan meist aus Acetat (acetoklastische Methanogenese) oder aus H₂ und CO₂ (hydrogenotrophe Methanogenese) gebildet. Als weitere Substrate sind auch niedermolekulare organische Verbindungen wie Methanol, Methylamine und Formiat bekannt. Im Zuge der hydrogenotrophen Methanogenese wird anorganischer Kohlenstoff mit Wasserstoff, entweder durch Substratoxidation gebildet oder aus der Umgebung aufgenommen, reduziert.

Methanogene Mikroorganismen (früher Methanbakterien) zählen zur Domäne der Archaeen (Archaea). Sie sind zur Methanbildung befähigt und können aus diesem Stoffwechselprozess Energie gewinnen. Diese Mikroorganismen werden in die Klassen Methanobacteria, Methanococci, Methanomicrobia und Methanopyri eingeteilt, zu denen insgesamt sechs Ordnungen gehören. H₂-oxidierende Methanbildner nutzen dabei die exergone Methanogenese, also die Reduktion von CO₂ mit molekularem Wasserstoff zu Methan und Wasser, als Energiequelle. Zu den obligat H₂-oxidierenden Methanogenen gehören z.B. die Gattungen Methanococcus, Methanobacterium und Methanopyrus. Es handelt sich dabei um strikt anaerobe Mikroorganismen, die bei Temperaturen zwischen 0 und 70°C aktiv sind. Generell steigt die Stoffwechselaktivität von Methanbildnern mit der Temperatur. Einige können auch bei Temperaturen größer 70-90°C überleben, wobei die Überlebensrate mit weiter steigenden Temperaturen abnimmt.

Die vorliegende Erfindung betrifft nun ein Verfahren zur hydrogenotrophen Methanogenese, d.h. die mikrobielle Bildung von CH₄ aus CO₂ und H₂, dadurch gekennzeichnet, dass ein Gasgemisch umfassend Erdgas, Wasserstoff und CO₂ mit einer Mindestkonzentration an Erdgas von 10 % und vorzugsweise einem für die Bildung von CH₄ stöchiometrischen Verhältnis zwischen Wasserstoff und CO₂ in einem eine Gaszone umfassenden untertägigen Speicher eingebracht und dort in Gegenwart von methanogenen Mikroorganismen gelagert wird. Die Herstellung des Wasserstoffs erfolgt vorzugsweise unter Verwendung erneuerbarer Energie, besonders bevorzugt direkt beim Speicher durch Elektrolyse von beispielsweise Wasser, wonach der so hergestellte Wasserstoff mit dem Erdgas bzw. bereits vorhandenen Erdgas/Wasserstoffgemischen vermischt und über eine Injektionsbohrung dem Speicher zugeführt wird. CO₂ stammt vorzugsweise aus einem der zuvor erwähnten Abtrennverfahren. Das umzuwandelnde CO₂ kann auch vor Zugabe von Wasserstoff mit dem Erdgas vermischt werden. Wenn der Wasserstoff durch Elektrolyse von Wasser hergestellt wurde, kann der als Abfallprodukt gewonnene Sauerstoff auch für die Herstellung von besonders reinem CO₂ verwendet werden. Die Gegenwart von Methanbildnern kann entweder durch Einbringen solcher Mikroorganismen in den Speicher, entweder vor oder nach Befüllen des Speichers, oder auch während der Befüllung des Speichers durch Zugabe der methanogenen Mikroorganismen in das einzubringende Gasgemisch sichergestellt werden. Alternativ kann die Zugabe von methanogenen Mikroorganismen auch unterbleiben, wenn diese im Speicher bereits in ausreichender Menge vorhanden sind. Überraschenderweise hat sich beim erfindungsgemäßen Verfahren gezeigt, dass bei Betrieb eines untertägigen Speichers, insbesondere eines untertägigen Erdgasspeichers, gleichzeitig mit dem normalen Gasspeicherbetrieb CO₂ und H₂ zu CH₄ umgewandelt werden kann. Durch das Verfahren der vorliegenden Erfindung kommt es gegenüber dem Stand der Technik zu wesentlichen Vorteilen, die aus der höheren Produktivität der methanogenen Mikroorganismen bei dem angegebenen, bestimmten Verhältnis zwischen Erdgas, Wasserstoff und CO₂ ersichtlich sind. Es kommt wider Erwarten trotz einer relativ hohen Konzentration von Erdgas im Zufuhrgas zu keiner Endprodukthemmung für die Methanogenese, die Anwesenheit des angegebenen Mindestanteils an Erdgas fördert sogar die Produktivität. Vorzugsweise beträgt der Mindestanteil an Erdgas mindestens 20 %, 30 %, 40 %, 50 %, 60 %, 70 %, 80 %, 87,5 %. Im Falle eines für die Bildung von CH₄ stöchiometrischen Verhältnisses zwischen Wasserstoff und CO₂ errechnet sich die Mindestmenge an Erdgas auch mit Werten, welche zwischen den oben angegebenen Mindestanteilen liegen, entsprechendes gilt auch für die im Folgenden angegebenen Mengen von Wasserstoff und CO₂. Durch das erfindungsgemäße Verfahren kann aus einem Abgas abgetrenntes CO₂ mit Hilfe von H₂ zu Erdgas umgewandelt werden, gemäß einer besonderen Ausführungsform ist es auch möglich, dass ein durch CO₂ verunreinigtes Erdgas direkt in seiner Lager- oder Speicherstätte gereinigt und von CO₂ befreit wird. In einem solchen Fall ist das Verhältnis zwischen Wasserstoff und CO₂ in dem einzubringenden Gasgemisch derart anzupassen, dass das nötige stöchiometrische Verhältnis zwischen Wasserstoff und CO₂ für die Bildung von CH₄ erst unter Hinzurechnung des in der Lagerstätte bereits vorhandenen, das gelagerte Erdgas verunreinigende CO₂ erreicht wird. Das gereinigte Erdgas bzw. CH₄ wird bei Bedarf über eine Entnahmebohrung aus dem Speicher entnommen.

Gemäß einer bevorzugten Ausführungsform der vorliegenden Erfindung ist diese dadurch gekennzeichnet, dass die Verweilzeit des Gasgemisches im untertägigen Speicher durch den Abstand zwischen Injektions- und Entnahmebohrung derart bestimmt wird, dass an der Entnahmebohrung Erdgas mit einem Anteil von unter 18 %, vorzugsweise unter 10 %, besonders bevorzugt unter 5 % des eingebrachten CO₂ entnommen wird. Überraschenderweise hat sich gezeigt, dass bereits nach wenigen Arbeitsphasen, also Zyklen des Umsatzes von CO₂ und H₂ mittels hydrogenotropher Methanogenese zu CH₄, die Reaktionsgeschwindigkeit der Umsetzung auch ohne Zugabe von Substrat so hoch ist, dass bei einem in Abhängigkeit von der Geologie des untertägigen Speichers gewählten geeigneten Abstands zwischen Injektions- und Entnahmebohrung die Umwandlung von CO₂ und H₂ mittels hydrogenotropher Methanogenese zu CH₄ kontinuierlich erfolgt, also über die Injektionsbohrung in den Speicher eingeleitetes CO₂ und H₂, bei Erreichen der Entnahmebohrung schon zur Gänze zu CH₄ umgewandelt ist. Wie in den nachfolgenden Beispielen gezeigt wird, beschleunigt sich die Arbeitsphase um das Dreifache und mehr, sobald sich im untertägigen Speicher eine ausreichende Population von methanogenen Mikroorganismen ausgebildet hat. Dabei hat sich auch gezeigt, dass entgegen der Lehre des Standes der Technik gemäß EP 0 963 780 A1 der für die Bildung von CH₄ nötige Wasserstoff nicht aus dem Porenwasser stammt, sondern aus dem zugesetzten gasförmigen H₂.

In der Geologie, Hydrogeologie und Bodenkunde bezeichnet die Porosität das Verhältnis des Volumens aller Hohlräume eines porösen Bodens oder Gesteins zu dessen äußerem Volumen. Es handelt sich um ein Maß dafür, wie viel Raum das Gestein aufgrund seiner Körnung oder Klüftung innerhalb eines bestimmten Volumens ausfüllt beziehungsweise welche Hohlräume es in diesem Volumen hinterlässt. Die Poren oder Kapillare sind dabei in der Regel mit Luft und/oder Wasser gefüllt. Diese Porosität (auch Porenraum genannt) bezieht sich auf das Volumen und wird üblicherweise in Prozent oder als Fraktion (Bruchteile von 1 = 100 %) angegeben und mit dem Formelbuchstaben φ bezeichnet.

Wenn der Anteil an Porenwasser im untertägigen Speicher vorzugsweise mindestens 15 % bezogen auf den Porenraum des Speichergesteins aufweist, hat sich überraschender Weise gezeigt, dass die Umwandlung von CO₂ und H₂ mittels hydrogenotropher Methanogenese zu CH₄ während des kontinuierlichen Betriebs des Speichers stattfinden kann, welcher dadurch definiert ist, dass die dem Speicher entnommene Gasmenge der in den Speicher eingebrachten Gasmenge entspricht. Eine bestimmte räumliche Entfernung zwischen Injektion- und Entnahmebohrung ist dabei nicht mehr nötig.

Gemäß einer bevorzugten Ausführungsform der vorliegenden Erfindung wird der pH in der Gaszone im Speicher durch dosierte Zugabe von Wasserstoff und/oder CO₂ gesteuert bzw. geregelt und in jenem Bereich gehalten, in welchem die Produktivität der methanogenen Mikroorganismen ein Optimum aufweist. Dieser Bereich variiert je nach vorhandener Mikroorganismenpopulation und liegt im Allgemeinen zwischen pH 5 und pH 10, vorzugsweise zwischen pH 6 und pH 9, besonders bevorzugt zwischen pH 7 und pH 8. Die Einstellung des pH-Wertes erfolgt dabei derart, dass bei zu hohem pH vermehrt CO₂ in die Gaszone zugeführt wird, wodurch sich der pH verringert, und bei zu geringem pH vermehrt molekularer Wasserstoff zugeführt wird, wodurch sich der pH erhöht. Gegebenenfalls kann die Zugabe von CO₂ und/oder Wasserstoff unter Auswertung der Daten einer sich in der Gaszone des Speichers befindlichen pH-Sonde zur Einhaltung eines vorgegebenen pH-Bereichs automatisiert werden.

Gemäß einer weiteren bevorzugten Ausführungsform der vorliegenden Erfindung werden die methanogenen Mikroorganismen mittels Inokulieren des Speichers durch Lagerstättenwasser aus einem anderen, sich bereits in Betrieb befindlichen Speicher angesiedelt. Das Lagerstättenwasser eines zweiten, sich bereits in Betrieb befindlichen Speichers hat sich dabei insofern als günstig erwiesen, als sich in diesem Lagerstättenwasser eine für die Methanisierung geeignete Mikroorganismenpopulation bereits ausgebildet hat. Nach dem Inokulieren vermehrt sich diese Population dann auch im neuen Speicher schneller als die Ausbildung einer eigenen Mikroorganismenpopulation dauern würde. Während des Wachstums im neuen Speicher wird sich die Zusammensetzung der Population dann auch gegebenenfalls ändern und an die vorhandenen Gegebenheiten neu anpassen, die Bakterienpopulation der Speicher stimmt dabei im Allgemeinen grundsätzlich in ihrer Zusammensetzung überein, v.a. hinsichtlich der zur hydrogenotrophen Methanogenese befähigten Archaeen kann sie sich jedoch unterscheiden, wobei die Unterscheidung zumeist in den prozentualen Anteilen besteht.

Als Beispiel für die Population in einem Speicher kann die mikrobielle Gemeinschaft in den im Zuge der Evaluierung des erfindungsgemäßen Verfahrens verwendeten Reaktoren herangezogen werden. Insgesamt wurden acht Reaktoren verwendet, das mikrobielle Konsortium aller verwendeten Reaktoren enthielt dabei die gleichen Genera.

In H₂ exponierten Reservoiren existieren Bakterien und Archaeen, die für mikrobielle Prozesse wie die Methanogenese, die Homoacetogenese, die Sulfat- und Eisenreduktion verantwortlich sind. Methanobacteriaceae ist die einzige bis heute bekannte Familie, die Methan aus Kohlendioxid oder -monoxid und Wasserstoff bilden kann. Zudem können sich die einzelnen Arten an einen weiten Bereich von Umweltbedingungen wie pH-Wert, Temperatur und Nährstoffverfügbarkeit anpassen. Das Genus Methanobacterium, bekannt für einen obligat anaeroben und chemolithotrophen Stoffwechsel, wurde zu einem geringen Anteil (0,3 - 1,2 %) in der mikrobiellen Gemeinschaft in den Reaktoren gefunden.

Thermotogaceae ist eine weitere wichtige Familie, die bekannt für ihre Fähigkeit zum Öl-Abbau ist. Sie ist in der mikrobiellen Gemeinschaft in den verwendeten Reaktoren durch die Genera Kosmotoga (gefunden in Anteilen von 1,7 - 6,4 %) und Petrotoga (gefunden in Anteilen von 2,4 - 6,9 %) vertreten. Beide Genera sind typisch für anaerobe Öl-Reservoire und kommen in einem breiten Temperaturbereich vor, von mesophil (20 - 45°C) bis thermophil (45 - 80°C). Sie sind als fermentative Bakterien beschrieben, die auch in der Lage sind elementaren Schwefel zu H₂S zu reduzieren. Weiters ist eine synergistische Wechselwirkung zwischen Methan-bildenden Bakterien und Wasserstoff-produzierenden Bakterien bekannt.

Eine weitere wichtige Familie, die Peptococcaceae (gefunden in Anteilen von 12,7 - 17,1 %), ist in der mikrobiellen Gemeinschaft in den Reaktoren beispielsweise durch folgende Genera vertreten: Desulfotomaculum (etwa 1,2 %), Desulfurispora (etwa 0,6 %), Dehalobacter (etwa 0,3 %) und Desulfitibacter (etwa 1,7 %) sowie weitere Peptococcaceae, die derzeit noch unkultiviert (14,03 - 7,92 %) sind. Desulfotomaculum sind mesophile obligat anaerobe Bakterien, die zur Sulfat-Reduktion und H₂S-Bildung befähigt sind. Man findet sie vorwiegend in mikrobiellen Biofilmen, in denen sie oft synergistische Konsortien mit fermentativen Bakterien bilden.

Die in der mikrobiellen Gemeinschaft der verwendeten Reaktoren ebenfalls aufgefundene Familie der Clostridiaceae (gefunden in Anteilen von 1,10 - 1,96 %) ist in der Lage, durch die Fermentation von Peptiden amorphes Eisen(III)oxidhydroxid zu reduzieren. Die Familie wird durch die Gattungen Natronincola und Proteiniclasticum vertreten. Weitere Bakterien, die in der Lage sind Eisen zu reduzieren und daher auch in der mikrobiellen Gemeinschaft eines Speichers bei Durchführung des erfindungsgemäßen Verfahrens vorkommen können, sind Shewanella, Desulfovibrio und Desulfuromonas, wobei diese Bakterien zusätzlich auch zur Reduktion von Mangan befähigt sind.

Die Thermoanaerobacteraceae, (gefunden in Anteilen von bis zu 5 %) vertreten durch Gelria (etwa 1,40 %), Moorella (etwa 2,35 %) und Syntrophaceticus (etwa 1,15 %), sind typisch für CO₂-reduzierende anaerobe Bedingungen. Für die Gattung Moorella sind mehrere Species bekannt, die Wasserstoff produzieren und ihn direkt an Methanbildner weiterleiten können.

Pseudomonas (gefunden in Anteilen von 14,26- 30,71 %) gehören zu einer Gruppe von Bakterien, die die höchste Vielseitigkeit bezüglich Metabolismus und Lebensraum aufweisen. *Pseudomonas stutzeri* beispielsweise kann unter anaeroben Bedingungen Nitrat zu Nitrit reduzieren und in weiterer Folge aus Nitrit molekularen Stickstoff bilden.

Wie bereits ausgeführt sehen die gesetzlichen Vorschriften in Österreich zurzeit eine maximale Wasserstoffkonzentration im Erdgas von 4 % vor, während in Deutschland beispielsweise 5 % zulässig sind. Die erfindungsgemäß vorgesehene Konzentration an Wasserstoff in dem zum Einspeisen in den Speicher vorgesehenen Gasgemisch kann daher in Abhängigkeit von den gesetzlichen Vorschriften des jeweiligen Lands unter 70 %, 40 %, 30 %, vorzugsweise unter 25 %, 24 %, 23 %, 22 %, 21 %, 20 %, 19 %, 18 %, 17 %, 16 %, 15 %, 14 %, 13 %, 12 %, 11 %, 10 %, 9 %, 8 %, 7 %, 6 %, 5 %, 4 %, 3 % bzw. unter 2 % betragen. Ein derartiges Gasgemisch (enthaltend beispielsweise in Österreich unter 4 % Wasserstoff im Erdgas) kann über die bestehende Infrastruktur des Gasnetzes transportiert werden und durch das erfindungsgemäße Verfahren dann während einer vergleichsweise kurzen Lagerung von Wasserstoff befreit werden, wobei gleichzeitig CO₂, beispielsweise als abgetrennter Bestandteil eines Abgases, in vorteilhafter Weise zu Methan umgewandelt wird. Durch das erfindungsgemäße Verfahren in Zusammenhang mit den verwendeten Speichern kann auch eine Vergleichmäßigung der fluktuierend anfallenden erneuerbaren Energie erzielt werden, indem durch mittels erneuerbarer Energie hergestellter Wasserstoff ins Erdgasnetz eingeschleust wird und dieser Wasserstoff dann im Bedarfsfall unter gleichzeitiger Verwertung von überschüssigem CO₂ im Speicher methanisiert werden kann.

Gemäß einer bevorzugten Ausführungsform der vorliegenden Erfindung ist vorgesehen, dass die Konzentration an CO₂ in dem zu lagernden Gasgemisch unter 20 %, vorzugsweise unter 19 %, 18 %, 17 %, 16 %, 15 %, 14 %, 13 %, 12 %, 11 %, 10 %, 9 %, 8 %, 7 %, 6 %, 5 %, 4 %, 3 %, 2 %, 1 % bzw. unter 0,5 % beträgt. Die Konzentration an CO₂ kann dabei durch Einspeisen von CO₂ in ein bereits in einem Speicher vorhandenes Erdgas/Wasserstoffgemisch vorgesehen werden, oder es kann durch das erfindungsgemäße Verfahren bei bekannter Konzentration an CO₂ in einem gespeicherten Erdgas durch Zugabe von mittels erneuerbarer Energie hergestelltem Wasserstoff in bestimmten Mengen nach der Reaktionsgleichung

CO₂+4H₂→CH₄+2H₂O

im Gasgemisch im Zuge der Speicherung ein gewünschter Gehalt an CO₂ eingestellt werden.

Beim erfindungsgemäßen Verfahren kann der Transport von Wasserstoff vorzugsweise im bereits bestehenden Erdgasnetz in einer den gesetzlichen Vorschriften entsprechenden Zumischung zum Erdgas erfolgen, die Lagerung des Erdgas/Wasserstoff/CO₂-Gemisches erfolgt vorzugsweise in unterirdischen Porenspeichem. Nach der Entnahme des gelagerten Gasgemisches aus der Lagerstätte und vor der Einspeisung in das Erdgasnetz kann das Gasgemisch dann gegebenenfalls noch einer Membrantrennung unterzogen werden, durch welche das Gasgemisch in Erdgas mit einem Wasserstoffgehalt entsprechend den gesetzlichen Vorschriften zum Einspeisen in das Erdgasnetz bzw. gewünschten falls in reines Erdgas einerseits und in ein Gasgemisch zur Rückführung in den Speicher andererseits aufgetrennt wird.

Beim erfindungsgemäßen Verfahren können vorzugsweise die methanogenen Mikroorganismen ausgewählt sein aus den Klassen Methanobacteria, Methanococci, Methanomicrobia und Methanopyri sowie Mischungen hiervon. Die Auswahl geeigneter methanogener Mikroorganismen liegt im Bereich des Wissens eines Fachmanns und hängt nicht zuletzt auch von den im Speicher bereits vorhandenen Mikroorganismen, den Umgebungsbedingungen im Speicher, dem Wassergehalt im Speicher sowie dem zu lagernden Gasgemisch ab. Gegebenenfalls können, wie bereits erwähnt, methanogene Mikroorganismen auch aus anderen, sich bereits in Betrieb befindlichen Speichern durch Einbringen der Bakterien in den neuen Speicher, entweder vor oder nach Befüllen des neuen Speichers, oder auch durch Einbringen der Mikroorganismen während der Befüllung des neuen Speichers durch Zugabe der methanogenen Mikroorganismen in das zu lagernde Gasgemisch vorgesehen werden.

Besonders bevorzugt ist der Speicher in dem erfindungsgemäßen Verfahren ein subterraner Porenspeicher. Vorstellbar ist auch eine Speicherung in Aquiferen, was aber einen größeren Aufwand sowohl von technischer als auch mikrobiologischer Seite bedeutet. Zum einen muss ein Aquiferspeicher mit hohem Druck betrieben werden und zum anderen befinden sich in einem Aquifer nicht dieselben Mikroben die in einer mit Kohlenwasserstoffen gesättigten Formation zu finden sind. Die Verwendung von künstlich geschaffenen Hohlräumen (Kavernen und Stollen) ist zumindest theoretisch ebenfalls möglich, obwohl hier die Oberfläche, an der sich ein Biofilm ausbilden kann, ungleich geringer ist als in einem Porenspeicher.

Gemäß einer weiteren Ausführungsform der vorliegenden Erfindung ist im Verfahren das Gasgemisch ein L-Gas. L-Gas (von engl. low (calorific) gas) ist ein Erdgas enthaltend bei Methananteilen von 80 bis 87 % neben dem Methan noch größere Mengen an Stickstoff und Kohlenstoffdioxid. Solches L-Gas kann durch das erfindungsgemäße Verfahren schnell und einfach in H-Gas (von engl. high (calorific) gas) mit einem Methangehalt von bis zu 99 % umgewandelt werden. Dabei kann bei einer vorhandenen Speicherstätte für L-Gas das erfindungsgemäße Verfahren derart aussehen, dass mittels erneuerbarer Energie hergestellter Wasserstoff, gegebenenfalls unter Beimischung von methanogenen Mikroorganismen wie oben angeführt, in die bestehende Lagerstätte eingebracht wird, wonach durch hydrogenotrophe Methanogenese aus vorhandenem CO₂ durch Verbrauch des zugesetzten Wasserstoffs sich in der Speicherstätte ein Erdgas mit hohem Methangehalt gebildet hat, welches gewünschten falls weder CO₂ noch Wasserstoff mehr enthält.

Genauso ist vorgesehen, dass das erfindungsgemäße Verfahren zur Steuerung des Gehalts von CO₂ und/oder Wasserstoff in einem Erdgas verwendet werden kann. Dazu kann entweder eine bekannte Menge an zugesetztem CO₂ durch methanogene Mikroorganismen unter Zugabe einer gegebenenfalls stöchiometrisch berechneten Menge an Wasserstoff während der Lagerung auf ein gewünschtes Maß reduziert werden, dies bei gleichzeitiger Bildung von Methan, oder es kann die gesamte Menge an CO₂ im gelagerten Gasgemisch durch Zugabe eines Überschusses an Wasserstoff entfernt werden, wobei das verbleibende Gasgemisch in der Lagerstätte gegebenenfalls bei Entnahme mittels Membrantrennung vom verbleibenden, überschüssigen Wasserstoff befreit werden kann.

Da auch aus Erdöllagerstätten sogenanntes Begleitgas mitgefördert wird, welches ebenfalls mit CO₂ beladen sein kann, ist gemäß einer bevorzugten Ausführungsform der vorliegenden Erfindung vorgesehen, die erfindungsgemäße Einbringung von Wasserstoff zur CO₂-Reduktion in Erdöllagerstätten einzusetzen. Dazu wird das erfindungsgemäße Verfahren zur Reduktion des CO₂-Anteils der Gaszone in einer natürlichen Erdgas- oder Erdöllagerstätte vor bzw. während der Förderung von Erdöl aus der Lagerstätte verwendet. Auch ist ein Hydrofracking von höheren Kohlenwasserstoffen sowie EOR dank Druckerhaltung als positiver Nebeneffekt vorstellbar.

In diesem Zusammenhang wird angemerkt, dass sowohl das genannte Begleitgas von Erdöllagerstätten als auch jegliche andere CO₂-hältige Gase, die im Zusammenhang mit dem Vorkommen bzw. der untertägigen Lagerung und/oder Speicherung von Erdöl oder Erdgas vorkommen, für den Zweck der vorliegenden Erfindung als unter den in der vorliegenden Beschreibung verwendeten Begriff Erdgas fallend angesehen werden.

Vor allem bei der neuen Implementierung des erfindungsgemäßen Verfahrens in einem bestehenden konventionellen Erdgasspeicher muss zumindest während der Initialisierung und/oder der ersten Arbeitsphasen damit gerechnet werden, dass die Verweilzeit im Untergrund nicht ausreicht, um den Wasserstoff und das CO₂ vollständig zu CH₄ umzusetzen. Dementsprechend kann beim erfindungsgemäßen Verfahren vorgesehen sein, einen Abscheider (Membran oder Keramik) am Gasausgang der Entnahmebohrung zu installieren und das dem Speicher entnommene Gas gemäß den normierten Spezifikationen aufzubereiten. Das dabei abgeschiedene, nicht umgesetzte CO₂/H₂-Gemisch kann dann wieder in den untertägigen Speicher re-injiziert und damit dem Umwandlungsprozess wieder zugeführt werden.

### Nachfolgende Beispiele erläutern die Erfindung:

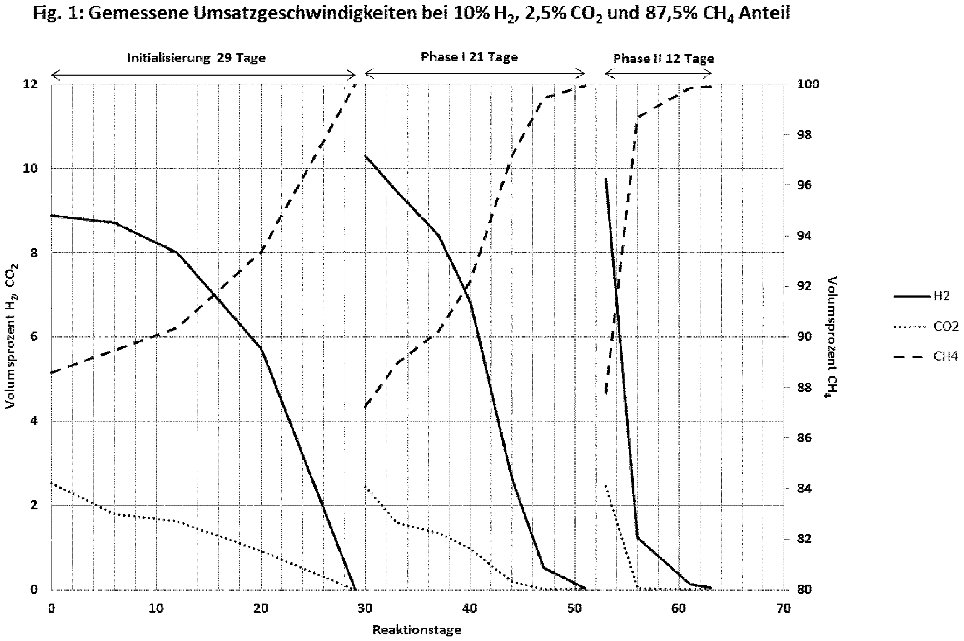

In insgesamt acht Bioreaktoren, die jeweils eine mikrobielle Gemeinschaft wie in der Beschreibung zuvor ausgeführt aufwiesen, wurde ein Gemisch aus CO₂ und H₂ eingeleitet, um mittels hydrogenotropher Methanogenese CH₄ zu gewinnen. Das eingeleitete Gasgemisch enthielt dabei 10 % H₂, 2,5 % CO₂ und 87,5 % CH₄, die Umwandlung des Gasgemisches wurde mittels Gaschromatographie detektiert. Die erhaltenen Ergebnisse werden in Figur 1 dargestellt.

Es konnte im Zuge der Versuche eindeutig bewiesen werden, dass sich mit der Anzahl der Arbeitsphasen (Befüllungszyklen) die Umwandlungszeit der Komponenten CO₂ und H₂ von anfänglich 29 Tagen für die Initialisierungsphase auf 12 Tage bei der zweiten Arbeitsphase reduzierte. Es wurde gefunden, dass der Grund dafür darin besteht, dass es einer gewissen Initialisierungszeit bedarf, in der sich die im Reaktor befindlichen Mikroorganismen an das eingebrachte Gasgemisch adaptieren. Während dieser Initialisierungszeit etablieren sich jene mikrobiellen Prozesse innerhalb einer Gemeinschaft von Mikroorganismen, die zur Verwertung von neu eingebrachten Substraten erforderlich sind. Zusätzlich erfolgt ein verstärktes mikrobielles Wachstum, i.e. die Zunahme der Anzahl an Substrat-verwertenden Mikroorganismen, welche dann ihrerseits das zugeführte Gasgemisch umwandeln, wodurch der Prozess maßgeblich beschleunigt wird.

Eine weitere Verkürzung der Arbeitsphasen ist im Dauerbetrieb sicherlich zu erwarten. Bezüglich der Initialisierungsphase ist bei großtechnischer Umsetzung des erfindungsgemäßen Verfahrens eine Lösung in der Betriebsführung denkbar, beispielsweise kann dem Gasgemisch im untertägigen Speicher die Verweilzeit gegeben werden, die es braucht, um sich vollständig umzuwandeln, oder es kann beispielsweise ein Umlaufbetrieb vorgesehen werden, bei dem mittels Membranen aus dem entnommenen Gas das überschüssige CO₂/H₂ abgeschieden wird. Nach momentanem Stand ist zu erwarten, dass sich nach 3-4 Arbeitsphasen das Idealsystem einstellen wird.

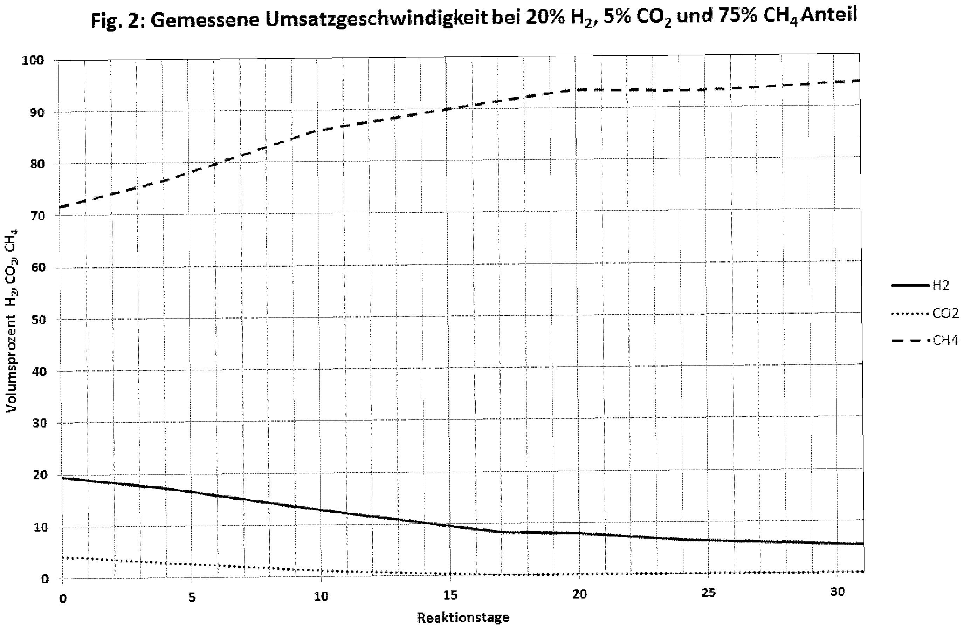

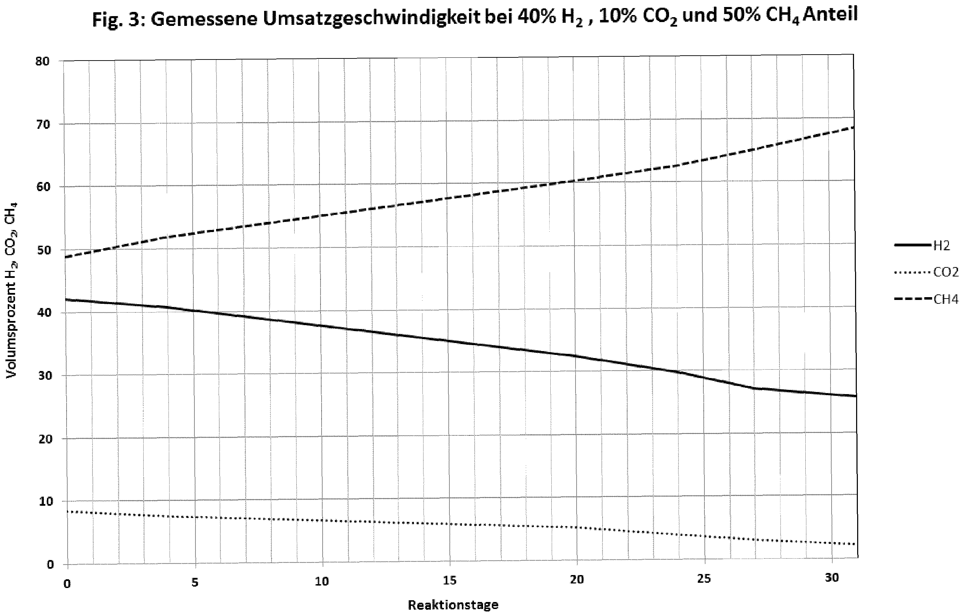

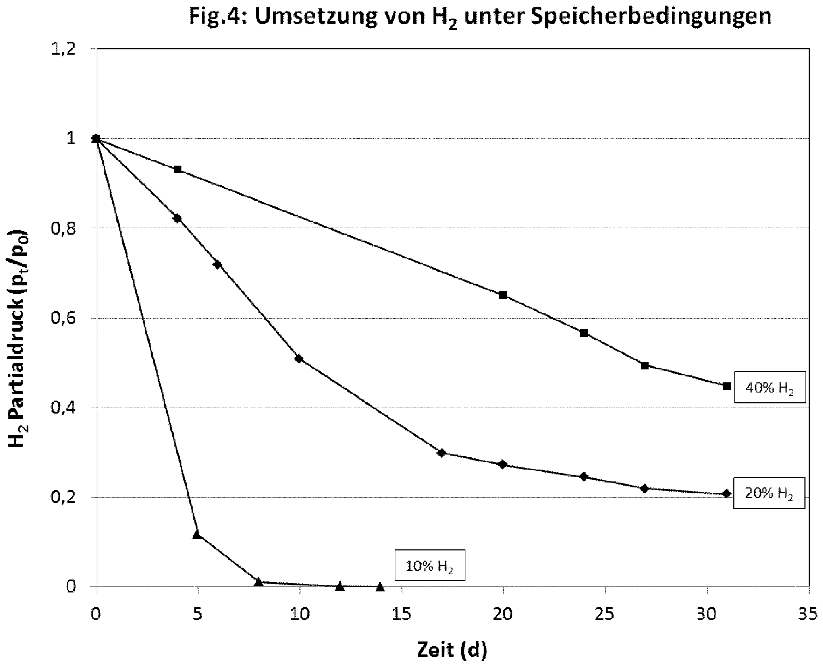

Bei den Versuchen gemäß Figur 2 und 3 wurden ebenfalls die oben erwähnten insgesamt acht Bioreaktoren verwendet, die jeweils eine mikrobielle Gemeinschaft wie in der Beschreibung zuvor ausgeführt aufwiesen. Gemäß Figur 2 wurde ein Gemisch aus 20 % H₂, 5 % CO₂ und 75 % CH₄ verwendet, gemäß Figur 3 ein Gemisch aus 40 % H₂, 10 % CO₂ und 50 % CH₄. Überraschenderweise hat ein höherer Wasserstoffgehalt im eingespeicherten Gas einen negativen Einfluss auf die Umsetzungsrate, wie sie sich aus dem linearen Bereich des Wasserstoffpartialdruckverlaufs nach Beginn des Experiments errechnete (Figur 4). So beträgt die Umsetzungsrate bei einem Anfangsgehalt von 10 % H₂ (2,5 % CO₂, 87,5 % CH₄) 0,54 bar/d, bei 20 % H₂ (5 % CO₂, 75 % CH₄) 0,44 bar/d und bei 40 % H₂ (10 % CO₂, 50 % CH₄) 0,34 bar/d. Bei Abwesenheit von Methan (80 % H₂, 20 % CO₂, nicht gezeigt) konnte überhaupt keine hydrogenotrophe Methanogenese beobachtet werden, woraus geschlossen werden kann, dass für das erfindungsgemäße Verfahren überraschenderweise die Anwesenheit von Methan im Ausgangsgasgemisch zwingend erforderlich ist.

## Patentansprüche

1. Verfahren zur hydrogenotrophen Methanogenese von H₂ und CO₂ zu CH₄, **dadurch gekennzeichnet, dass** ein Gasgemisch umfassend Erdgas, Wasserstoff und CO₂ mit einer Mindestkonzentrationen an Erdgas von 10 Vol. % und vorzugsweise einem für die Bildung von CH₄ stöchiometrischen Verhältnis zwischen Wasserstoff und CO₂ in einem eine Gaszone umfassenden untertägigen Speicher eingebracht und dort in Gegenwart von methanogenen Mikroorganismen gelagert wird.

2. Verfahren nach Anspruch 1, **dadurch gekennzeichnet, dass** die Verweilzeit des Gasgemisches im untertägigen Speicher durch den Abstand zwischen Injektions- und Entnahmebohrung derart bestimmt wird, dass an der Entnahmebohrung Erdgas mit einem Anteil von unter 18 Vol. %, vorzugsweise unter 10 %, besonders bevorzugt unter 5 % des eingebrachten CO₂ entnommen wird.

3. Verfahren nach Anspruch 1 oder 2, **dadurch gekennzeichnet, dass** der untertägige Speicher einen Wasseranteil von mindestens 15 % bezogen auf den Porenraum des Speichergesteins aufweist und die Umwandlung während des kontinuierlichen Betriebs des Speichers stattfindet.

4. Verfahren nach einem der Ansprüche 1 bis 3, **dadurch gekennzeichnet, dass** der pH in der Gaszone im Speicher durch dosierte Zugabe von Wasserstoff und/oder CO₂ gesteuert bzw. geregelt und in jenem Bereich gehalten wird, in welchem die Produktivität der methanogenen Mikroorganismen ein Optimum aufweist.

5. Verfahren nach einem der Ansprüche 1 bis 4, **dadurch gekennzeichnet, dass** die methanogenen Mikroorganismen mittels Inokulieren des Speichers durch Lagerstättenwasser aus einem anderen, sich bereits in Betrieb befindlichen Speicher angesiedelt werden.

6. Verfahren nach einem der Ansprüche 1 bis 5, **dadurch gekennzeichnet, dass** die Konzentration an Wasserstoff in dem zum zu lagernden Gasgemisch unter 72 Vol. %, vorzugsweise unter 40 %, besonders bevorzugt unter 30 %, 20 % 13 %, 12 %, 11 %, 10 %, 9 %, 8 %, 7 %, 6 %, 5 %, 4 %, 3 % bzw. unter 2 % beträgt.

7. Verfahren nach einem der Ansprüche 1 bis 6, **dadurch gekennzeichnet, dass** die Konzentration an CO₂ in dem zu lagernden Gasgemisch unter 18 Vol. %, vorzugsweise unter 10 %,9 %, 8 %, 7 %, 6 %, 5 %, 4 %, 3 %, 2 %, 1 % bzw. unter 0,5 % beträgt.

8. Verfahren nach einem der Ansprüche 1 bis 7, **dadurch gekennzeichnet, dass** Bakterien ausgewählt aus den Klassen Methanobacteria, Methanococci, Methanomicrobia und Methanopyri sowie Mischungen hiervon verwendet werden.

9. Verfahren nach einem der Ansprüche 1 bis 8, **dadurch gekennzeichnet, dass** der untertägige Speicher ein Porenspeicher ist.

10. Verwendung des Verfahrens nach einem der Ansprüche 1 bis 9 zur Reduktion des CO₂-Anteils der Gaszone in einer natürlichen Erdgas- oder Erdöllagerstätte vor bzw. während der Förderung von Erdöl aus der Lagerstätte.

11. Verwendung eines Verfahrens nach einem der Ansprüche 1 bis 9 zur Steuerung des Gehalts von CO₂ und/oder Wasserstoff in einem Erdgas.

## Claims

1. Method for the hydrogenotrophic methanogenesis of H₂ and CO₂ to form CH₄, **characterized in that** a gas mixture comprising natural gas, hydrogen generated and CO₂ having a minimum natural gas concentration of 10 vol% and preferably having a ratio of hydrogen and CO₂ that is stoichiometric for the formation of CH₄ is fed into an underground storage facility which comprises a gas zone and is stored there in the presence of methanogenic microorganisms.

2. Method according to claim 1, **characterized in that** the residence time of the gas mixture in the underground storage facility is determined by the distance between injection and withdrawal bore such that natural gas having a content of less than 18 vol%, preferably less than 10 %, particularly preferably less than 5 %, of the injected CO₂ is withdrawn at the withdrawal bore.

3. Method according to claim 1 or 2, **characterized in that** the underground storage facility has a water content of at least 15 % of the pore space of the reservoir rock and the conversion occurs during the continuous operation of the storage facility.

4. Method according to any one of claims 1 to 3, **characterized in that** the pH value in the gas zone of the storage facility is regulated by means of a controlled addition of hydrogen and/or CO₂ and is kept within a range that is optimal with respect to the productivity of the methanogenic microorganisms.

5. Method according to any one of claims 1 to 4, **characterized in that** the methanogenic microorganisms are provided by means of inoculating the storage facility with deposit water from other storage facilities that are already in operation.

6. Method according to any one of claims 1 to 5, **characterized in that** the hydrogen concentration in the gas mixture to be stored is lower than 72 vol%, preferably lower than 40 %, particularly preferably lower than 30 %, 20 %, 13 %, 12 %, 11 %, 10 %, 9 %, 8 %, 7 %, 6 %, 5 %, 4 %, 3 % or lower than 2 %.

7. Method according to any one of claims 1 to 6, **characterized in that** the CO₂ concentration in the gas mixture to be stored is lower than 18 vol%, preferably lower than 10 %, 9 %, 8 %, 7 %, 6 %, 5 %, 4 %, 3 %, 2 %, 1 % or lower than 0.5 %.

8. Method according to any one of claims 1 to 7, **characterized in that** bacteria selected from the classes of Methanobacteria, Methanococci, Methanomicrobia and Methanopyri as well as mixtures thereof are used.

9. Method according to any one of claims 1 to 8, **characterized in that** the underground storage facility is a pore storage facility.

10. Use of the method according to any one of claims 1 to 9 for reducing the CO₂ content in the gas zone of a naturally occurring fossil oil or natural gas deposit, either before or after the extraction of fossil oil from the deposit.

11. Use of a method according to any one of claims 1 to 9 for regulating the CO₂ and/or hydrogen content in a natural gas.

## Revendications

1. Procédé pour la méthanogenèse hydrogénotrophe de H₂ et de CO₂ en CH₄, **caractérisé en ce qu'**un mélange gazeux comprenant du gaz naturel, de l'hydrogène et du CO₂, présentant des concentrations minimales de gaz naturel de 10 % en volume et de préférence un rapport stoechiométrique, pour la formation de CH₄, entre l'hydrogène et CO₂, est introduit dans un réservoir souterrain comprenant une zone gazeuse, et y est stocké en présence de microorganismes méthanogènes.

2. Procédé selon la revendication 1, **caractérisé en ce que** le temps de séjour du mélange gazeux dans le réservoir souterrain est déterminé par la distance entre le forage d'injection et le forage de prélèvement de façon à prélever au niveau du forage de prélèvement un gaz naturel présentant une proportion du CO₂ introduit inférieure à 18 % en volume, de préférence inférieure à 10 %, d'une manière particulièrement préférée inférieure à 5 %.

3. Procédé selon la revendication 1 ou 2, **caractérisé en ce que** le réservoir souterrain présente une proportion d'eau d'au moins 15 % par rapport au volume de pores de la roche réservoir, la conversion ayant lieu pendant l'exploitation continue du réservoir.

4. Procédé selon l'une des revendications 1 à 3, **caractérisé en ce que** le pH dans la zone gazeuse se trouvant dans le réservoir est commandé ou réglé par un addition dosée d'hydrogène et/ou de CO₂, et est maintenu dans la plage dans laquelle la productivité des microorganismes méthanogènes présente un optimum.

5. Procédé selon l'une des revendications 1 à 4, **caractérisé en ce que** les microorganismes méthanogènes sont colonisés par inoculation du réservoir par de l'eau de gisement provenant d'un autre réservoir, se trouvant déjà en exploitation.

6. Procédé selon l'une des revendications 1 à 5, **caractérisé en ce que** la concentration de l'hydrogène dans le mélange gazeux à stocker est inférieure à 72 % en volume, de préférence inférieure à 40 %, d'une manière particulièrement préférée inférieure à 30 %, 20 %, 13 %, 12 %, 11 %, 10 %, 9 %, 8 %, 7 %, 6 %, 5 %, 4 %, 3 %, ou inférieure à 2 %.

7. Procédé selon l'une des revendications 1 à 6, **caractérisé en ce que** la concentration du CO₂ dans le mélange gazeux à stocker est inférieure à 18 % en volume, de préférence inférieure à 10 %, 9 %, 8 %, 7 %, 6 %, 5 %, 4 %, 3 %, 2 %, 1 % ou inférieure à 0,5 %.

8. Procédé selon l'une des revendications 1 à 7, **caractérisé en ce que** les bactéries sont choisies dans les classes Methanobacteria, Methanococci, Methanomicrobia et Methanopyri, ainsi que les mélanges de ceux-ci.

9. Procédé selon l'une des revendications 1 à 8, **caractérisé en ce que** le réservoir souterrain est un réservoir en couche poreuse.

10. Utilisation du procédé selon l'une des revendications 1 à 9 pour la réduction de la proportion de CO₂ de la zone gazeuse dans un gisement naturel de gaz naturel ou de pétrole, avant ou pendant la production de pétrole à partir du gisement.

11. Utilisation d'un procédé selon l'une des revendications 1 à 9 pour commander la teneur d'un gaz naturel en CO₂ et/ou en hydrogène.
